(19) **Europäisches Patentamt European Patent Office Office européen des brevets**

(11) **EP 4 374 806 B1**

(12) **EUROPEAN PATENT SPECIFICATION**

(45) Date of publication and mention
of the grant of the patent:
**02.10.2024  Bulletin 2024/40**

(21) Application number: **22461631.8**

(22) Date of filing: **23.11.2022**

(51) International Patent Classification (IPC):
**A61B 17/62** $^{(2006.01)}$     **A61B 6/04** $^{(2006.01)}$
**G16H 20/40** $^{(2018.01)}$     **A61B 17/66** $^{(2006.01)}$

(52) Cooperative Patent Classification (CPC):
**A61B 17/62; A61B 17/66; G16H 20/40;**
A61B 6/0421; A61B 6/0442; A61B 6/0492;
A61B 2090/061; A61B 2090/3966; G16H 40/67

(54) **ORTHOPAEDIC DEVICE AND SYSTEM FOR MANAGING THERAPY**

ORTHOPÄDISCHE VORRICHTUNG UND SYSTEM ZUR THERAPIEVERWALTUNG

DISPOSITIF ORTHOPÉDIQUE ET SYSTÈME DE GESTION DE THÉRAPIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**29.05.2024  Bulletin 2024/22**

(73) Proprietor: **Mazurkiewicz, Piotr
31-762 Krakow (PL)**

(72) Inventors:
• **Mazurkiewicz, Piotr
31-762 Krakow (PL)**

• **Karpiel, Grzegorz
31-841 Krakow (PL)**
• **Prusak, Daniel
31-621 Krakow (PL)**

(74) Representative: **Bury & Bury
Ul. Przyczolkowa 124
02-968 Warszawa (PL)**

(56) References cited:
WO-A1-2008/002992     CN-A- 114 053 014
US-A1- 2016 022 314     US-A1- 2022 071 662

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Field of the invention

The invention concerns an orthopaedic device and system for managing therapy especially of broken bones.

## Background of the invention

A method of orthopaedic device fixation based on image analysis, especially Roentgen images, is known from the publication of the international patent application WO2011146703. Elements of the device, visible on X-rays images, are used to reconstruct a three-dimensional representation of the position and/or orientation of a first and a second segment of a bone in relation to the orthopaedic device.

Disclosed method of fixation relies on a registration from two different directions, a first and a second image of the orthopaedic device, together with segments of the bone and creating a transformation matrix for both images by means of a computer system and reconstructing of a three-dimensional model (3D) of the segments of the bone with the orthopaedic device on a basis of the elements of the orthopaedic device and the segments of the bone identified in the images.

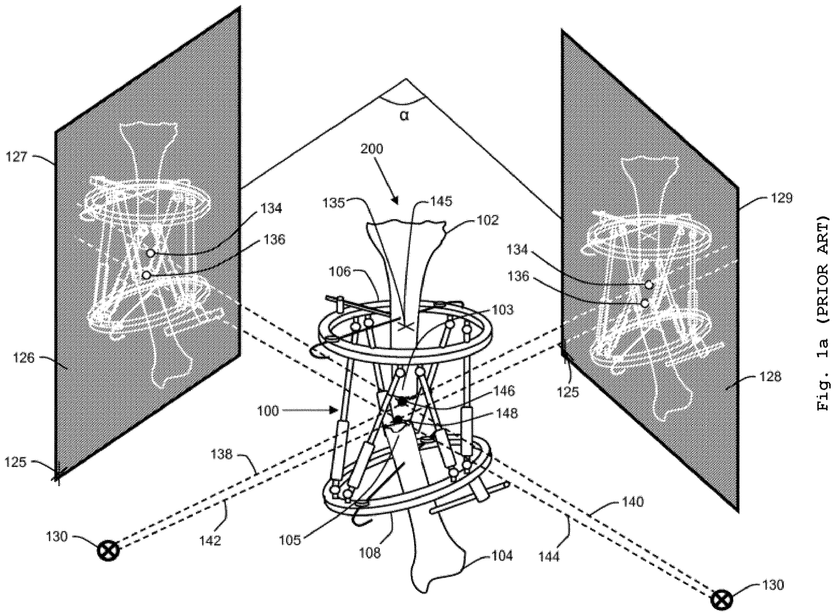

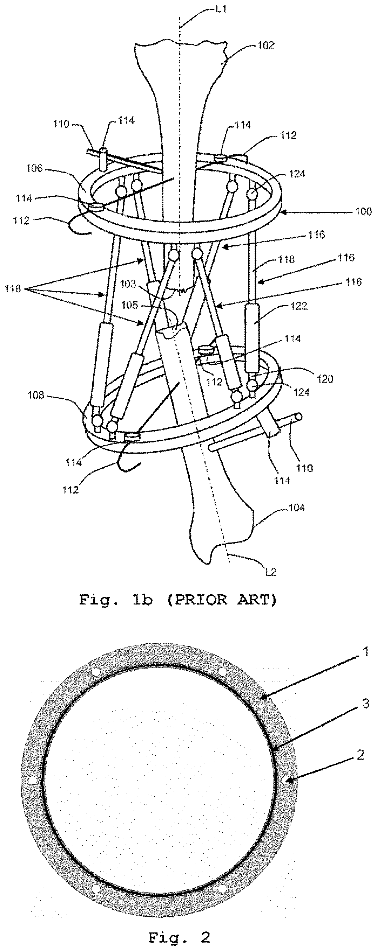

The device, according to WO2011146703, consists of two fixator rings connected by spacing elements of adjustable length, so called distractors. As shown in Fig. 1a and b, the device comprises an upper fixator ring **106** and a lower fixator ring **108** connected by spacing elements realized as distractors **116.** The distractors are provided with joints **124.** At other mounting points, wires and nails are mounted to the rings, which connect the device with the bones. The orthopaedic device is mounted on the affected limb of the patient and is set so to ensure proper bone fusion. The orthopaedic device is provided with the markers visible in X-ray images and mounted in well-defined positions, in relation to other elements of the orthopaedic device. Known arrangement of the markers facilitates creating of the transformation matrix and reconstruction of a three-dimensional model of the segments of the bone with the orthopaedic device. Using markers with well-defined shapes facilitates their identification in the images.

It is possible to provide patient with instruction how to regulate the settings of distractors in the process of healing. Known solutions involve two dimensional (time×distractor) table of subsequent settings for every distractor, which is quite complicated. Regrettably physicians have limited possibilities of remotely controlling progress in healing of the broken bone and verification, if settings applied by the patients are correct - therefore multiple visits are required.

A disadvantage of the device, known in the state-of-the-art, is that the markers are not always well visible and easy to recognize in the X-ray images, this can be a source of errors and makes automatization of 3D modelling of the device, regulation and positioning based on this model difficult. In particular, by using various markers, despite the fact that various shapes are used, it is not always possible to distinguish them in the image.

Publication of international patent application no WO2008002992 discloses an improvement - a radiolucent external fixation element with embedded radio-opaque alignment structure in form of a wire. The alignment structure is used to form a plane orthogonal to the axis of the bone and center the fixation element upon the axis of the bone. Such solution enhances positioning of the fixation element with X-Ray. Wire radio opaque alignment structure may be deformed during regulation of the orthopaedic device, during therapy. The structure with embedded ring is either less durable under stress (if made of metal) or difficult to process for movable joints. US2016022314A1 discloses an external bone fixation device configured to correct bone deformities or repair bone injuries. The device can include a plurality of bases configured to be attached to portions of a bone and a plurality of struts configured to be adjustable in length to change the position and orientation of the plurality of bases and the attached bone portions. US2022071662A1 discloses a strut measurement and feedback device and corresponding systems and methods for use with an external fixator are disclosed. US2022071662A1 discloses an orthopaedic device has a first fixator ring and a second fixator ring coupled via at least three adjustable spacing elements. Each of the first and the second fixator rings has means for connecting the adjustable spacing elements. Each adjustable spacing element has a tube and an externally threaded rod having a first end coupled to the first fixator ring and a second end that protrudes to the first end of the tube and is movable inside the tube. The tube has second end coupled to the second fixator ring. Each of the at least three adjustable spacing elements is provided with a first joint for connecting the externally threaded rod to the first fixator ring and a second joint for connecting the tube with the second fixator ring. The first and the second joints are fixedly connected to the first and the second fixator rings respectively. The tube has a longitudinal slit, and machine readable scale provided on its outer surface readable by position reading device slidably attached to the tube. The strut measurement and feedback device can provide real-time feedback to an individual as the length of the strut is being adjusted to ensure the length adjustment complies with a prescription for the length of the strut. As a result, a patient can more effectively comply with the prescription as adjustments to the strut are made over time, thereby improving the likelihood of successful bone alignment.

## Summary of the invention

The objective of the invention is to solve above problems and provide orthopaedic device durable, reliable and easy for positioning with X-rays. Further, it is an object of the invention to provide orthopaedic device having two fixator rings coupled via adjustable spacing elements on the basis of X-ray images and enabling autonomous regulation of the adjustable spacing elements by the patient - according to instruction prepared by the doctor - in a safe and controlled manner.

An orthopaedic device has the first fixator ring and the second fixator ring coupled via at least three adjustable spacing elements. The first and the second fixator ring are made of material at least partially permeable for X-radiation and have means for connecting spacing elements, a marker located inside the ring and made of a material visible in images made by means of X-radiation, having a form of arc segment, made of a material of lower X-radiation transmission factor than an average transmission factor of the fixator ring material. Arcs are easy to detect and precise positioning on images taken with X-radiation. The fixator rings further have means for connecting the spacing elements. Adjustable spacing element has a tube and an externally threaded rod, having a first end coupled to the first fixator ring and a second end, that protrudes to the first end of the tube and is movable inside the tube. The tube has the second end coupled to the second fixator ring.

The adjustable spacing element is provided with a first rotation compensating joint for connecting the externally threaded rod to the first fixator ring and a second rotation compensating joint for connecting the tube with the second fixator ring. The first and the second rotation compensation joint are fixedly connected to the first and the second fixator rings, respectively. The tube has an inner element with internally threaded portion compatible with the thread of the externally threaded rod. In that configuration the patient can regulate the length of spacing element by rotating the whole tube which is easy and convenient. The rotation compensable joints prevent the fixator rings from being subjected to stress and damage or deformation when the spacing element is manually rotated. As the joints are fixedly attached to the fixator rings, there is no need to weaken the fixator rings, to provide movable/rotatable connection.

The tube further has a longitudinal slit with machine readable scale provided on its outer surface. The orthopaedic device further has a position reading device movably attached to the tube. The position reading device has a power supply, processing unit with memory, communication means and scale reader unit for reading machine readable scale of the tube, and is coupled with the second end of the externally threaded rod via the slit. The position reading device enables distribution the absolute length of the spacing element to the remote device, e.g. patient mobile device or external server. That in turn enables control of tuning of lengths of the spacing elements applied by the patient and providing him directions. As the position reading device is individually assigned to the distractor, the problem of two dimensional table of settings is alleviated. Accordingly, every distractor may have a setting list with time stamps loaded into memory for guiding user with respect to required settings via communication means. Communication means may include user interface means for communicating with the user directly or electronic communication means for guiding user via an app on external device e.g. smartphone. This configuration of measurement allows using relatively thin tube and avoid shielding important details when X-ray images are taken.

Advantageously, the machine readable scale is a visible pattern provided on an external surface of the tube and the reader unit is optical reading unit. That kind of scale can be read with simple CCD sensor.

Alternatively, the machine readable scale is a magnetic pattern provided on an external surface of the tube and the reader unit is magnetic reading unit. Such scale is less affected by dirt.

Advantageously, at least one of the first rotation compensating joint and the second rotation compensating joint is a spherical joint.

Alternatively, at least one of the first rotation compensating joints and the second rotation compensating joint is an universal joint with additional bearing providing rotation.

Advantageously, the first end of the tube has securing sleeve movable from unlocked position to locked position wherein the internally threaded portion is cleft and has the thread compatible with the externally threaded rod when sleeve is in locked position and expanded when the sleeve is in unlocked position.

Advantageously, the position reading device has user interface means for providing an information to the user. Using the user interface means it is possible to give direction to the patient regarding position reading devices without any remote devices - directly. Successive tuning instructions can be loaded to the memory of the position reading device, interpreted by the processing unit and compared against absolute reading of the position from reader unit. User interface communication means may include diodes, buzzers or soft vibrations. Alternatively communication means may be used for communicating with user via his device.

A system for managing therapy according to the invention comprised of at least one orthopaedic device according to the invention and communication data hub, server and terminal. Position reading devices are connected via communication links with the communication data hub which is connected with the server via communication network. Terminal is connected to the server via communication network. The server is adapted to receive from communication data hub positions read by position reading devices and then send said positions to the terminal. Server is also adapted to communicate adjustment settings from the terminal to the respective position reading devices via data communication hub.

## Short description of figures

The invention is described in more detail below with reference to its embodiments presented in the attached drawings in which Fig. 1 a and b present an orthopaedic device known in the state-of-the-art, Fig. 2 presents a fixator of a device according to the invention with a marker close to its inner edge, Fig. 3 presents a magnified fragment of the ring illustrating exemplary way of placement of the marker, Fig. 4 presents a ring, according to an alternative embodiment with two breaks in perspective partial section, Fig. 5 shows fixator with the marker located close to the external edge of the fixator, Fig. 6a and 6b present a medical device, according to the invention, Fig. 7a and b show a tube of adjustable spacing element, Fig. 8 shows a block diagram of the system according to the invention.

## Description of embodiments of the invention

The fixator ring **1** shown in Fig. 2 has a substantially circular shape. It is made of composite with glass fibres, so of semi-permeable material for X-radiation. The fixator ring is provided with holes constituting mounting points **2** enabling connection with spacing elements with regulated length - so called distractors - and with elements used to connect medical devices with patient's bones.

The fixator ring **1** is provided with a marker constituting an arc segment **3** in the form of a circle made of a titanium wire, low permeable to X radiation. Thanks to that, the fixator ring **1** is semi-permeable, the marker is well visible in X-rays images taken with the use of X radiation. In those image, the circle marker constituting the arc segment **3** in the form of the circle due to projecting being represented in the form of ellipses with different parameters. Those ellipses are easy to recognize, also algorithmically, and on a basis of parameters of the ellipses in two images taken from different directions which determine a plane situated at an angle to the plane, on which a segment, having a form of the circle, is placed, translation matrices can be determined and a position of the arc segment **3** in the space can be reconstructed. Because the marker in the form of the arc segment **3** is rigidly fixed to the fixator ring **1,** determining the position and the orientation of the marker enables to determine the position and the orientation of the whole fixator ring **1.**

Circular marker integrated in a ring has significant advantages over the typical markers attached to the fixator rings. Firstly it need not be initially set and then removed. Secondly it need not be in certain position - only taking images from two orthogonal directions is required.

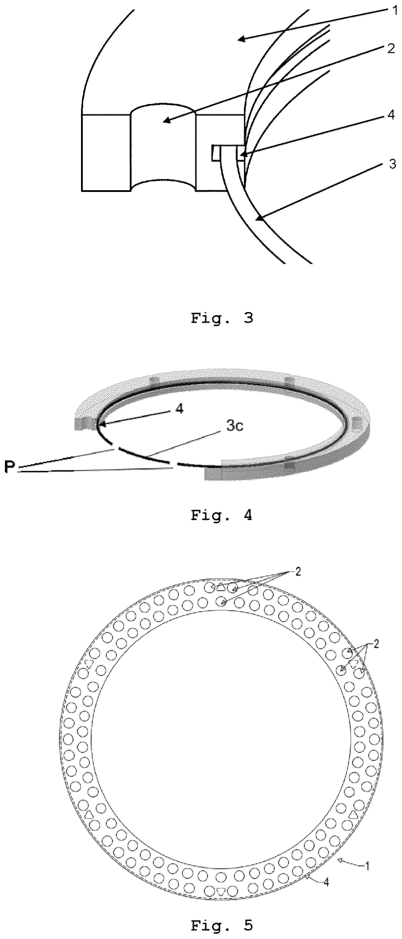

The arc segment **3** in the form of the circle segment can be sunk in the ring, however it can be technologically troublesome - in particular when a freedom of arranging markers with breaks is desired. An alternative is to provide the fixator ring with profiled or milled canal **4** dedicated to receive the arc segment **3.** Such solution is visible in Fig. 3, presenting the embodiment, in which the arc segment **3** is placed in the canal **4** provided in the inside of the fixator ring **1.** In such configuration placing the marker in the ring is easy, it comes down to wire winding by its guidance in the canal **4.** The canal **4** can be then durably sealed.

In a case of using an aluminium ring **1** and a steel arc segment **3,** cold welding can be used, which enables bonding in a permanent way.

Imaging is convenient, when the absorption coefficient in the wavelength range of X-radiation of the material of which the arc segment **3** is made is at least twice higher than an absorption coefficient of which the fixator ring is made. Then it is easier to distinguish the marker in the images by not changing the gamma correction used in imaging of the bones. Nevertheless, advantageous properties of the invention are provided, even using the arc segment **3** made of titanium in the fixator ring **1** made of aluminium.

A bigger difference in the absorption coefficients are ensured by aluminium and stainless steel. The aluminium fixator ring **1** with the arc segment **3** in the form of the circle made of stainless steel wire is easy to manufacture and is well recognizable in the X-ray images.

Good results of the experiments were obtained by using the rings made of aluminium, a composite of the carbon fibres, a composite of the glass fibres, an aluminium composite with the carbon fibres and an aluminium composite with carbon fibres and glass fibres. On the background of those materials, the markers in the form of the arc segment made of the wire made of titanium, silver, stainless steel or carbon steel were clearly visible. All those materials performed well in medical applications.

The marker **3** constituting the arc segment with at least one break **P** enables easier determination of the orientation in space. Using more markers enables determination and identification of many rings in the image by distribution of sections and breaks. An example of the fixator ring comprising the marker in the form of the arc segment with two breaks **P** is illustrated in Fig. 4. If two fixator rings provided with markers in the form of intermittent arc segments are visible in the image, and each of those markers has breaks of different lengths, it is very easy to distinguish rings in the image, as well as to match the orientation of the ring **1** in space. Fig. 5 shows top view of a fixator ring with marker **4** located next to external edge.

The experiments shown, that there is no need for using the whole closed ring. Good results were obtained even for the markers in the form of the arc segment of the length 1/16 of the ring circumference. Using the arc segment partially opened, can be used for obtaining an additional information about the position. Simultaneously, a significant fragment of the whole arc is necessary in order to reduce errors of determining transformation matrix, mapping the shape of the marker in reality onto the shape in X-rays images. Ranges of ¼ to ¾ length of circumference of the whole ring were particularly comfortable.

Marker in the form of the arc segment placed on the circle has such advantage, that in the mathematical operations of image projection on the plane, the circles and their arcs can be relatively straightforward mapped onto ellipses and segments of the ellipses in the image plane. The circle needs to be regular not deformed though. Consequently it is easier to match the model to the real contour in the image. As a rule, the longer the segment, the smaller the matching error and as a consequence smaller error of determining the projection parameters. The markers of other shapes can be used, suitable for placing in the fixator ring. The ring and the marker arranged on concentric circles constitutes the simplest computational example. However, the markers and the fixator rings of other shapes can be used - in particular elliptical. It is good when those shapes can be easily described with mathematical equations and distributed on the ring. Therefore the ellipses are the natural candidates.

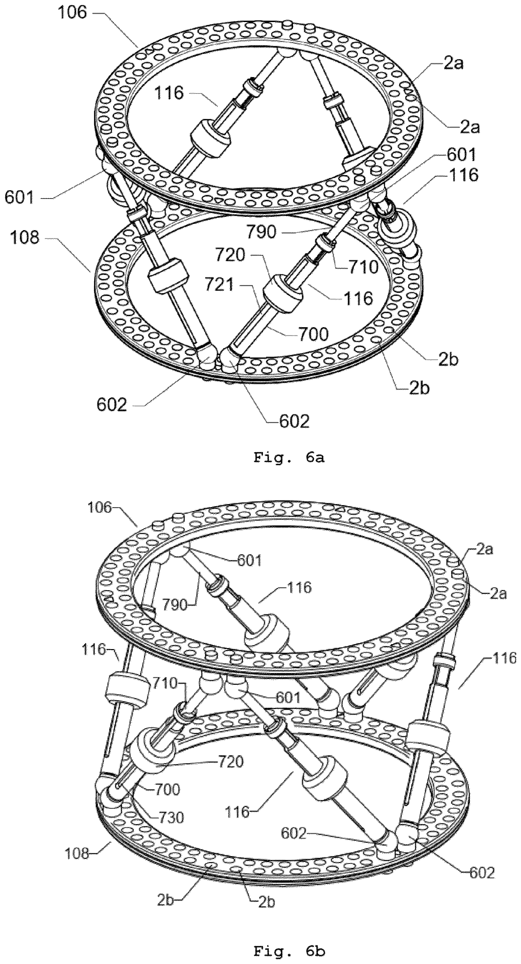

In Fig. 6a and 6b an embodiment of a device, according to the invention, is shown in different perspective views. The device comprises the first fixator ring **106** and the second fixator ring **108,** connected with at least three spacing adjustable elements - distractors **116** connected with rings at mounting points **2a** and **2b**. The mounting points constitute holes. The mounting points of different types adapted to receive mounting means can be used, distractors, wires, nails and other accessories useful in the application of the device.

Due to ergonomics of handling of the orthopaedic device comprising two fixator rings **106, 108** connected by adjustable distractors **116,** the possibility of mounting distractors near the outer part of the fixator ring **106, 108** should be provided. Therefore, holes constituting mounting points **2a** and **2b** are provided on the outside edge. Therefore, in order to obtain better results, it is better to arrange marker on the inside of the fixator ring **106, 108,** so that its image will not be distorted by the images of the mounting points.

Thanks to using the markers in the form of the arc segments, the position and the orientation of the fixator ring can be reconstructed in the wide range of angles. Convenient for numerical reasons and due to the simplicity of manufacturing are markers in the form of circles made of silver wire, placed in the structure of the fixator ring, which in this situation is also circular in shape. Sinking of the arc segment **3** in the form of the wire in the circular fixator ring **106, 108** has such effect, that the position and the orientation of the arc segment **3** are substantially equivalent to the position and the orientation of the fixator ring **106, 108.**

The position and the orientation of the marker in the form of the arc segment **3** can be determined on the basis of at least two images: a first image taken by means of X-rays radiation and a second image taken by the means of X-rays radiation. In order to improve accuracy and reliability, more images taken from different angles can be used.

In this embodiment, the shape of the upper arc segment **3** is described by the equation:

$$a(x,y): (x - x_a)^2 + (y - y_a)^2 = R_a^2 \ ,$$

and the lower arc segment

$$b(x,y): (x - x_b)^2 + (y - y_b)^2 = R_b^2 \ ,$$

in which for simplicity, it is assumed $(x_a, y_a) = (x_b, y_b) = (0,0)$, whereas $R = R_a = R_b$ constitutes known radius of the arc segments circle **3.** Naturally, different arc segments can be used in the upper and the lower ring. They can differ in size as well in shape and the number of breaks. Such solution allows for easy algorithmically distinction of the fixator rings **106, 108** and to determine how they are oriented in relation to the bones visible in the X-rays images.

By analysing the position of the arc segment - for example, the upper arc segment **3** - for all images a processing stage is carried out, in which they are displayed to the operator, allowing him to indicate on the computer screen the point located within the upper arc segment **3**. The operator, by means of the indicating device, indicates at least 5 points belonging to the mapping of the upper arc segment **3** in the image. A larger number of the points can be assumed in order to obtain better accuracy. The lower arc segment is handled analogously.

Thanks to using a method described above, initial conditions can be determined for regulating the orthopaedic device comprising two fixator rings connected with distractors **116.** Such initial conditions constitute positions and orientation of the fixator rings and settings of the distractors **116.** The setting of the distractor **116** is its length - in solutions in which the distractors **116** with adjustable length are used.

Lengths of the distractors need to be adjusted in a process of healing. Use of fixator rings with embedded markers bring up certain problems. Fixator rings with a slit for marker are prone to deformation or damaging due to mechanical stress. Composite fixators are difficult to process and therefore convenient mounting points for distractors are just holes **2,2a,2b** for connecting distractors **116** fixedly to fixator rings.

The position of the fixator rings and distractors' settings **116** is determined on a basis of at least a first image taken with X-ray radiation and the second image taken with X-ray radiation. In a situation, in which it is not possible to obtain the mapping of the arc segments **3** of both fixator rings in the one image, more image should be taken. If both markers are visible in each image, then the user can determine separately five points in the first image, belonging to the mapping of the arc segment **3** of the upper fixator ring **106** and separately five points belonging to the mapping of the marker **3** of the lower fixator ring **108.**

Having five points for each of the markers in two images, the position and the orientation of the first fixator ring **106** and the second fixator ring **108** can be determined algorithmically.

Perspective views of an embodiment of a device according to the invention are shown in Fig. 6a and 6b. Knowing to which mounting points **2a** and **2b,** the distractors **116** are mounted, their desirable initial length is determined and a therapy is prescribed, which consists of gradually modifying the length of the distractors **116** in accordance with the desired medical effect.

Adjustable spacing element **116** has a tube **700** and an externally threaded rod **790** having a first end coupled to the first fixator ring **106** and a second end that protrudes to the first end **710** of the tube **700** and is movable inside the tube **700**. The tube **700** has second end coupled to the second fixator ring **106**. The adjustable spacing element **116** is provided with a first rotation compensating joint **601** for connecting the externally threaded rod **790** to the first fixator ring **106** and a second rotation compensating joint **602** for connecting the tube with the second fixator ring **108**. The first and the second rotation compensation joints **601, 602** are fixedly connected to the first and the second fixator rings **106, 108,** respectively. The tube **700** has an internally threaded portion **711** compatible with the thread of the externally threaded rod **790**.

That allows adjusting the length of the distractor **116** by rotating whole tube **700**. This however requires rotation compensation on joints **601** and **602**. It can be obtained with spherical joint which enables tilting the distractors and compensation on rotation. Alternatively a universal joint (so called cardan joint) can be used in combination with additional bearing allowing free rotation of the tube **700** and rod **790** along their longitudinal axis.

This additional bearing advantageously can be configured to allow only step rotation blocking the rotation in fixed position. That reduces risk of self de-regulation of the mechanism when patient walks. Additionally allowing quantized regulation and/or giving the user clicking sound corresponding to number of steps makes regulation more convenient. It has been observed that 24 to 60 clicks per rotation allow easy regulation and still prevent the mechanism from uncontrolled rotation when patient walks.

The tube **700** has longitudinal slit **730** allowing for coupling the second end of the rod **790** located inside the tube **700** with the position reading device **720** located in a casing surrounding the tube **700.** and machine readable scale **721** provided on its outer surface. The position reading device **720** is movably attached to the tube **700,** and can move along it with the second end of the rod **790**. Therefore absolute position of the rod **790** with respect to the tube **700** can be read from the scale **721**. Accordingly position reading device **720** reads absolute length of the distractor **116**.

The position reading device has a power supply e.g. battery or solar cell. It feeds energy to processing unit which has a memory, communication means for communicating with other devices and scale reader unit for reading machine readable scale **721** of the tube **700**. The processing unit follows a program load into the memory and computes absolute length of the distractor **116** based on a readout from the scale reader unit. Processing unit delivers results to external devices e.g. mobile device of the patient or external server available for the doctor supervising the treatment of the patient. Accordingly the treatment is under control and directions can be given to the patient via his mobile device to adjust the lengths of the distractors **116** when required.

The instructions can be individually loaded to the memory of position reading devices **720** in a form of a list with time stamps. Providing position reading devices **720** with interface means for communication with patient allows then convenient adjustment of length according to individual predefined instruction comprising setting and time stamps or even instructions tuneable by physician. Convenient interface are diodes indicating when the tubes **700** need to be rotated and by how much. Additionally attention of the user can be attracted with buzzer indicating that there is a time to adjust and alarming when adjustment goes out of limit. Accordingly neither patient nor doctor has to bother with 2D table - it is enough just to apply rotation to distractor until it signals that it is ready.

If process of healing goes faster or slower it is possible for the physician to adjust just time stamps in every distractor without a need to rebuilt 2D table known in the art.

The scale **721** is a visible pattern provided on an external surface of the tube **700** and the reader unit optical reading unit e.g. CCD reader. Alternatively the machine readable scale can be invisible or transluscent for human eye, e.g. a magnetic pattern provided on an external surface of the tube **700** and the reader unit is magnetic reading unit can be applied. The visible scale is easier to make but magnetic is less susceptible to dirt.

The invention is much safer than known automatic regulating devices as the patient adjusts distractors by himself but under control of the device. Therefore in case of any error the pain or the signal from position reading device will prevent patient form doing harm. The latter is not an option with the automatically adjustable devices. The invention also controls absolute position of the scale reader unit with respect to scale as opposed to dead reckoning (e.g. counting turns) known in the art. That allows avoiding accumulation of measurement errors and make the treatment safer.

Additionally, measurement equipment and automatic regulation devices are known to limit the range of adjustment. The present invention allows using simple mechanical constructions providing wide range of adjustment and making the therapy more efficient in terms of use of distractors.

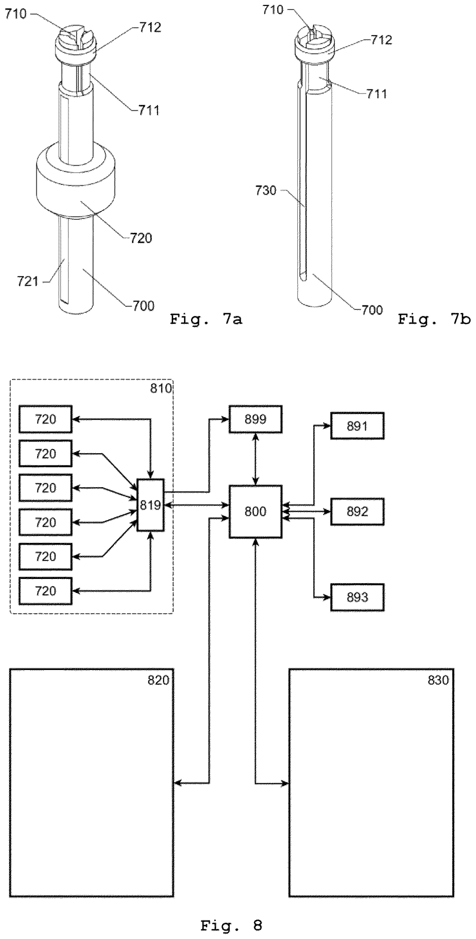

In the embodiment shown in Fig. 7a and b the first end **710** of the tube **700** of distractor has securing sleeve **712** movable from unlocked position to locked position. The internally threaded portion **711** located at the cleft end **710** of the tube **700**. The threaded portion **711** is located at cleft section of the tube **700**. The internal thread of the portion **711** is compatible with the externally threaded rod when sleeve **712** is in locked position (shown in Fig. 7a). When the sleeve **712** is moved to unlocked position the cleft end **710** widens, internally threaded portion **711** widens and the external thread of the externally threaded rod is let go. That allows setting initial position easily by the doctor the locking the device and allowing patient only for adjusting by rotation according to instructions - the treatment plan.

The invention uses individual devices for measuring length of distractors and therefore reduces risk of a mistake in a long treatment process involving setting applied by the a person. This approach also makes it easier for physicians to correct the treatment plan and in combination with communication means it makes it possible to do it remotely. Furthermore use of integrated markers make it easier to calibrate the system and to control the process using the same marker setup for subsequent X-rays.

The invention allows easy scaling up the system and organize therapies for multiple patients in convenient system. An example of such system is shown in Fig. 8. The system has a central server **800** with terminal devices **891, 892, 893,** via which one or more of physicians can set up and monitor therapy for their patients. The system, by way of example, comprises three orthopaedic devices **810, 820, 830** used to treat three different patients. Configuration of the system is discussed with respect to the first orthopaedic device **810**. Device has additional data hub **819,** communicating with server **800** via communication network e.g. wirelessly via IEEE802.11 or via cellular network such as GSM, LTE or 5G. The communication data hub communicates with position reading devices **720.** Preferably and conveniently it is wireless communication e.g. Bluetooth, or Bluetooth Low Energy. The server **800** is adapted to receive from communication data hub **819** positions read by position reading devices **720** and then send said positions to the terminal **891, 892, 893.** The server is further adapted to communicate adjustment settings from the terminal **891, 892, 893** to the respective position reading devices **720** via data communication hub **819.** Accordingly the system allows centralized management of therapy and in case of emergency providing diagnosis data to physician managing the therapy or his replacement.

For convenience of the user, it is possible to provide communication between his mobile device **899** e.g smartphone and the data hub, for better visualization of therapy progress and instructions with respect to adjustments. Alternatively user terminal can be connected to the server but with different privileges level.

The embodiments of the invention discussed above should not be interpreted as limiting the scope of the invention, but merely as the embodiments which facilitates its understanding. In particular, a number, a type and a character of the mounting points and distractors can be different from the embodiment. The person skilled in the art, having read this description, is also able to select other materials, of which the ring and the marker can be made, ensuring the visibility of the marker in the ring and making suitable for medical applications.

**Claims**

1. An orthopaedic device **(810, 820, 830)** having a first fixator ring **(106)** and a second fixator ring **(108)** coupled via at least three adjustable spacing elements **(116),** wherein each of the first and the second fixator rings **(106, 108)** is made of material at least partially permeable for X-radiation and has means for connecting adjustable spacing elements **(116),**

   has a marker located inside the ring and made of a material visible in images made by means of X-radiation, having a form of arc segment **(3, 3c),** made of a material of lower X-radiation transmission factor than an average transmission factor of the fixator ring material and has means **(2)** for connecting the adjustable spacing elements **(116),**
   each of the at least three adjustable spacing elements **(116)** has a tube **(700)** and an externally threaded rod **(790)** having a first end coupled to the first fixator ring **(106)** and a second end that protrudes to the first end **(710)** of the tube **(700)** and is movable inside the tube, wherein the tube has second end coupled to the second fixator ring **(106),**
   **wherein**
   each of the at least three adjustable spacing elements **(116)** is provided with

   a first rotation compensating joint **(601)** for connecting the externally threaded rod **(790)** to the first fixator ring **(106)** and
   a second rotation compensating joint **(602)** for connecting the tube **(700)** with the second fixator ring **(108),** wherein

   the first and the second rotation compensation joints **(601, 602)** are fixedly connected to the first and the second fixator rings **(106, 108),** respectively
   the first end **(710)** of the tube **(700)** is cleft and the tube **(700)** has an internally threaded portion **(711)** which at the cleft of the tube **(700)** has an internal thread compatible with the thread of the externally threaded rod **(790),** a longitudinal slit **(730),** and machine readable scale **(721)** provided on its outer surface,

each tube (700) further having a position reading device (720) movably attached thereto, wherein the position reading devices (720) have a power supply, a processing unit with memory, communication means and a scale reader unit for reading the machine readable scale of the tube, and are coupled with the second end of the respective externally threaded rods (790) via the longitudinal slits **(730).**

2. Orthopaedic device **(810, 820, 830)** according to claim 1, wherein the machine readable scale **(721)** is a visible pattern provided on an external surface of the tube **(700)** and the reader unit optical reading unit.

3. Orthopaedic device **(810, 820, 830)** according to claim 1, wherein the machine readable scale is a magnetic pattern provided on an external surface of the tube **(700)** and the reader unit is magnetic reading unit.

4. Orthopaedic device **(810, 820, 830)**according to claim 1, 2 or 3, wherein at least one of the first rotation compensating joint **(601)** and the second rotation **(602)** compensating joint is a spherical joint.

5. Orthopaedic device **(810, 820, 830)** according to claim 1, 2 or 3, wherein at least one of the first rotation compensating joint and the second rotation compensating joint is an universal joint with additional bearing.

6. Orthopaedic device **(810, 820, 830)**according to any one of claims 1 to 5, wherein the first end **(710)** of the tube **(700)** has securing sleeve **(712)** movable from an unlocked position to a locked position, wherein the internally threaded portion **(711)** is cleft and has the thread compatible with the externally threaded rod **(790)** when sleeve is in the locked position and expanded when the sleeve is in the unlocked position.

7. Orthopaedic device **(810, 820, 830)** according to any one of claim 1 to 6, wherein the communication means of position reading device **(720)** include user interface means for providing an information to the user.

8. Orthopaedic device **(810, 820, 830)** according to claim 7, wherein the output means comprise a buzzer.

9. Orthopaedic device **(810, 820, 830)** according to claim 8, wherein the output means comprise a light emitting diode.

10. A system for managing therapy comprising at least one orthopaedic device **(810, 820, 830)** as defined in claim 1, further comprising a data communication hub **(819)**, a server **(800)** and a terminal **(891, 892, 893)**, wherein the position reading devices **(720)** are connected via communication links with the communication data hub **(819)** which is connected with the server **(800)** via a communication network wherein the terminal **(891, 892, 893)** is connected to the server via a communication network, and the server **(800)** is adapted to receive from the communication data hub **(819)** positions read by the position reading devices **(720)** and then send said positions to the terminal **(891, 892, 893)** and communicate adjustment settings from the terminal **(891, 892, 893)** to the respective position reading devices **(720)** via the data communication hub **(819).**

**Patentansprüche**

1. Orthopädische Vorrichtung **(810, 820, 830)** mit einem ersten Fixierring **(106)** und einem zweiten Fixierring **(108),** welche Fixierringe durch zumindest drei einstellbaren Abstandshaltern **(116)** miteinander verbunden sind, wobei jeder der ersten und zweiten Fixierringe **(106, 108)**

aus einem Material gefertigt ist, welches zumindest teilweise durchlässig für Röntgenstrahlen ist und welcher Mittel zur Befestigung von einstellbaren Abstandshaltern **(116)** aufweist, einen Marker umfasst, welcher Marker innerhalb des Rings angeordnet ist, und aus einem Material besteht, welches in Röntgenbildern sichtbar ist, und welcher Marker die Form eines Bogensegments **(3, 3c)** aufweist, und aus einem Material besteht, welches einen geringeren Transmissionsfaktor für Röntgenstrahlung aufweist als der durchschnittliche Transmissionsfaktor des Materials des Fixierrings, und Mittel **(2)** zur Verbindung der einstellbaren Abstandshalter **(116)**

aufweist, wobei jeder der zumindest drei einstellbaren Abstandshalter **(116)** ein Rohr **(700)** und einen Gewindestab **(790)** mit einem Aussengewinde umfasst, welcher Gewindestab ein erstes Ende, verbunden mit dem ersten Fixierring **(106)** und ein zweites, sich zu dem ersten Ende **(710)** des Rohrs **(700)** ersteckendes, Ende aufweist, welches zweite Ende des Gewindestabs innerhalb des Rohrs bewegbar ist und welches Rohr ein zweites Ende aufweist, dass mit dem zweiten Fixierring **(108)** verbunden ist, wobei

jeder der zumindest drei Abstandshaltern **(116)** ausgerüstet ist mit

einem ersten rotationskompensierenden Gelenk **(601)** zur Verbindung des Gewindestabs **(790)** mit dem ersten Fixierring **(106)** und
einem zweiten rotationskompensierenden Gelenk **(602)** zur Verbindung des Rohrs **(700)** mit dem zweiten Fixierring **(108),** wobei

das erste und zweite rotationskompensierende Gelenk **(601, 602)** fixierbar mit dem ersten und zweiten Fixierring **(106, 108)** verbunden sind, das erste Ende **(710)** des Rohrs **(700)** gespalten ist, und wobei das Rohr **(700)**

ein Innengewinde **(711)**, welches an der Spaltung des Rohrs **(700)** ein mit dem Aussengewinde des Gewindestabs **(790)** kompatibles Gewinde ausbildet,
einen Längsschlitz **(730)**, und
eine maschinenlesbare Skalierung **(721),** angeordnet an der Aussenseite des Rohrs umfasst,

wobei jedes Rohr **(700)** zudem Vorrichtungen zur Bestimmung der Position **(720)** umfasst, welche Vorrichtungen zur Bestimmung der Position **(720)** bewegbar an dem Rohr angebracht sind und eine Energieversorgung, eine Steuereinrichtung mit Speichermitteln, Kommunikationsschnittstellen und eine Ablesevorrichtung zum Ablesen der maschinenlesbaren Skalierung umfassen, und welche Vorrichtungen zur Bestimmung der Position durch den Längsschlitz **(730)** mit dem zweiten Ende eines Gewindestabs **(790)** in Verbindung bringbar sind.

2. Orthopädische Vorrichtung **(810, 820, 830)** nach Anspruch 1, wobei die maschinenlesbare Skalierung **(721)** als ein sichtbares Muster auf der Aussenfläche des Rohrs **(700)** und die Vorrichtung zur Bestimmung der Position als eine optische Vorrichtung ausgebildet ist.

3. Orthopädische Vorrichtung **(810, 820, 830)** nach Anspruch 1, wobei die maschinenlesbare Skalierung als ein magnetisches Muster auf der Aussenfläche des Rohrs **(700)** und die Vorrichtung zur Bestimmung der Position als eine magnetische Vorrichtung ausgebildet ist.

4. Orthopädische Vorrichtung **(810, 820, 830)** nach Anspruch 1, 2 oder 3, wobei zumindest eines des ersten rotationskompensierenden Gelenks **(601)** und des zweiten rotationskompensierenden Gelenks **(602)** ein Kugelgelenk ist.

5. Orthopädische Vorrichtung **(810, 820, 830)** nach Anspruch 1, 2 oder 3, wobei zumindest eines des ersten rotationskompensierenden Gelenks und des zweiten rotationskompensierenden Gelenks ein Kreuzgelenk mit zusätzlicher Lagerung ist.

6. Orthopädische Vorrichtung **(810, 820, 830)** nach einem der Ansprüche 1 bis 5, wobei das erste Ende **(710)** des Rohrs **(700)** eine Sicherung **(712)** umfasst, welche Sicherung von einer ungesicherten in eine gesicherte Position bewegbar ist, wobei das Innengewinde **(711)** gespalten ist und, insofern sich die Sicherung in der gesicherten Position befindet, ein Gewinde ausbildet, dass mit dem Aussengewinde des Gewindestabs **(790)** kompatibel ist und, insofern sich die Sicherung in der ungesicherten Position befindet, expandiert wird.

7. Orthopädische Vorrichtung **(810, 820, 830)** nach einem der Ansprüche 1 bis 6, wobei die Kommunikationsschnittstellen der Vorrichtung zur Bestimmung der Position **(720)** Informationsschnittstellen umfassen, um für den Anwender der Vorrichtung Informationen bereitzustellen.

8. Orthopädische Vorrichtung **(810, 820, 830)** nach Anspruch 7, wobei die Ausgabeschnittstelle ein Vibrationselement umfasst.

9. Orthopädische Vorrichtung **(810, 820, 830)** nach Anspruch 8, wobei die Ausgabeschnittstelle eine lichtemittierende Diode umfasst.

10. Ein System zur Therapiekontrolle umfassend mindestens eine orthopädische Vorrichtung **(810, 820, 830)** gemäss Anspruch 1, zusätzlich umfassend eine Datenkommunikationsknoten **(819)**, einen Server **(800)** und eine Bedieneinheit **(891, 892, 893)**, wobei die Vorrichtung zur Bestimmung der Position **(720)** über Kommunikationsschnittstellen mit dem Datenkommunikationsknoten **(819)** verbunden ist, welcher Datenkommunikationsknoten **(819)** über eine Netzwerkverbindung mit dem Server **(800)** verbunden ist und wobei die Bedieneinheit **(891, 892, 893)** über eine Netzwerkbindung mit dem Server **(800)** verbunden ist, und der Server derart einrichtbar ist, dass

er Positionen, welche von der Vorrichtung zur Bestimmung der Position **(720)** aufgenommen wurden, von dem Datenkommunikationsknoten empfängt und diese Positionen an die Bedieneinheit **(891, 892, 893)** sendet und
Korrektureinstellungen von der Bedieneinheit **(891, 892, 893)** via des Datenkommunikationsknotens **(819)** an die jeweilige Vorrichtung zur Bestimmung der Position **(720)** übermittelt.

**Revendications**

1. Dispositif orthopédique **(810, 820, 830)** ayant un premier anneau de fixation **(106)** et un deuxième anneau de fixation **(108)** couplé via au moins trois éléments d'espacement réglables **(116),** chacun des premier et deuxième anneaux de fixation **(106, 108)**

   étant constitué d'un matériau au moins partiellement perméable aux rayons X et comportant des moyens de raccordement pour les éléments d'écartement réglables **(116),**
   possédant un marqueur situé à l'intérieur de l'anneau constitué en un matériau visible sur des images réalisées au moyen de rayons X, ayant une forme de segment d'arc **(3, 3c),** réalisé en un matériau dont le facteur de transmission de rayons X inférieur au facteur de transmission moyen du matériau de l'anneau de fixation et étant pourvu de moyens **(2)** de raccordement d'éléments d'écartement réglables **(116),**
   chacun au moins des trois éléments d'écartement réglables **(116)** comportant un tube **(700)** et une tige filetée **(790)** dont une première extrémité est couplée au premier anneau de fixation **(106),** et dont une deuxième extrémité fait saillie vers la première extrémité **(710)** du tube **(700)** et est déplaçable à l'intérieur du tube, le tube ayant une deuxième extrémité couplée au deuxième anneau de fixation **(108),**
   chacun au moins des trois éléments d'écartement réglables **(116)** étant muni

   d'un premier joint de compensation en rotation **(601)** pour le raccordement de la tige filetée **(790)** au premier anneau de fixation **(106)** et
   un deuxième joint de compensation en rotation **(602)** pour le raccordement du tube **(700)** avec le deuxième anneau de fixation **(108),**

   le premier et le deuxième joints de compensation en rotation **(601, 602)** étant respectivement connectés de manière fixe aux premier et deuxième anneaux de fixation **(106, 108),**
   la première extrémité **(710)** du tube **(700)** étant fendue et le tube **(700)** ayant

   une partie taraudée **(711)** qui est pourvue d'un taraudage compatible avec le filetage de la tige filetée **(790)** au niveau de la fente du tube **(700),**
   une fente longitudinale **(730),** et
   une échelle lisible par machine **(721)** prévue sur sa surface externe,

   chaque tube **(700)** ayant en outre un dispositif de lecture de position **(720)** fixé de manière mobile, les dispositifs de lecture de position **(720)** étant pourvus d'une alimentation électrique, d'une unité de traitement dotée d'une mémoire, de moyens de communication et d'une unité de lecture de l'échelle pour lire l'échelle lisible par machine du tube, et étant couplés à la deuxième extrémité des tiges filetées respectives **(790)** via les fentes longitudinales **(730).**

2. Dispositif orthopédique **(810, 820, 830)** selon la revendication 1, l'échelle lisible par machine **(721)** étant un motif visible aménagé sur une surface externe du tube **(700)** et de l'unité de lecture optique.

3. Dispositif orthopédique **(810, 820, 830)** selon la revendication 1, l'échelle lisible par machine étant un motif magnétique aménagé sur une surface externe du tube **(700),** et l'unité de lecture étant une unité de lecture magnétique.

4. Dispositif orthopédique **(810, 820, 830)** selon la revendication 1, 2 ou 3, au moins un parmi le premier joint compensateur de rotation **(601)** et le deuxième joint de compensation en rotation **(602)** étant un joint sphérique.

5. Dispositif orthopédique **(810, 820, 830)** selon la revendication 1, 2 ou 3, au moins un parmi le premier joint compensateur de rotation et le deuxième joint de compensation en rotation étant un joint universel avec un palier supplémentaire.

6. Dispositif orthopédique **(810, 820, 830)** selon l'une des revendications 1 à 5, la première extrémité **(710)** du tube **(700)** ayant un manchon de fixation **(712)** déplaçable depuis une position déverrouillée vers une position verrouillée, la partie taraudée **(711)** étant fendue et ayant un pas de filetage compatible avec celui de la tige filetée **(790)** lorsque le manchon se trouve dans la position verrouillée et élargi lorsque le manchon est dans la position déverrouillée.

7. Dispositif orthopédique **(810, 820, 830)** selon l'une des revendications 1 à 6, les moyens de communication du dispositif de lecture de position **(720)** incluant une interface utilisateur pour fournir une information à l'utilisateur.

8. Dispositif orthopédique **(810, 820, 830)** selon la revendication 7, les moyens de sortie comprenant un avertisseur sonore.

9. Dispositif orthopédique **(810, 820, 830)** selon la revendication 8, les moyens de sortie comprenant une diode électroluminescente.

**10.** Système de gestion de thérapie comprenant au moins un dispositif orthopédique **(810, 820, 830)** tel que défini dans la revendication 1, comprenant en outre un centre de communication de données **(819),** un serveur **(800)** et un terminal **(891, 892, 893),** les dispositifs de lecture de position **(720)** étant connectés via des liens de communication avec le centre de communication de données **(819)** qui est connecté avec le serveur **(800)** via un réseau de communication dans lequel le terminal **(891, 892, 893)** est connecté au serveur via un réseau de communication, et le serveur **(800)** étant adapté pour

recevoir du centre de communication de données **(819)** les positions lues par les dispositifs de lecture de position **(720)** et ensuite envoyer lesdites positions au terminal **(891, 892, 893)** et communiquer des réglages d'ajustement du terminal **(891, 892, 893)** aux dispositifs de lecture de position **(720)** respectifs via le centre de communication de données **(819)** .

Fig. 1a (PRIOR ART)

Fig. 1b (PRIOR ART)

Fig. 2

Fig. 3

Fig. 4

Fig. 5

Fig. 6a

Fig. 6b

Fig. 7a

Fig. 7b

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011146703 A
- WO 2008002992 A
- US 2016022314 A1
- US 2022071662 A1